# EUROPEAN PATENT APPLICATION

(11) **EP 4 464 273 A1**
(43) Date of publication of application: **20.11.2024**
(21) Application number: 24154884.1
(22) Date of filing: 31.01.2024
(51) Int. Cl.: A61B 90/50, A61B 8/00, F16M 11/04

(54) **GRAVITY COMPENSATION CONTROL DEVICE AND ULTRASONIC DIAGNOSTIC DEVICE INCLUDING THE SAME**

(30) Priority: 15.05.2023 KR 20230062486
(71) Applicant: Samsung Medison Co., Ltd., Hongcheon-gun, Gangwon-do 25108 (KR)
(72) Inventor: Kim, Yeonho, 05340 Seoul (KR); Sim, Sungtae, 05340 Seoul (KR); Cha, Jinwoo, 05340 Seoul (KR)
(74) Representative: Frey, Sven Holger

(57) **Abstract**

Provided is a gravity compensation control device including a first mounting unit, a rotatable link, one end of which is connected to a compensation shaft arranged in the first mounting unit and another end of which is vertically movable by a weight applied thereto, wherein a portion between the ends is supported by a fixed shaft arranged in the first mounting unit, such that the link is rotatable about the fixed shaft, and a weight compensation device, wherein the weight compensation device includes a bearing unit including a bearing shaft arranged in the first mounting unit, and a bearing coupled to the bearing shaft, an auxiliary link connected to the compensation shaft and one side of the bearing unit, and an elastic member coupled to another side of the bearing unit and arranged in a lengthwise direction of the auxiliary link.

## Description

### CROSS-REFERENCE TO RELATED APPLICATION

This application is based on and claims priority under 35 U.S.C. §119 to Korean Patent Application No. 10-2023-0062486, filed on May 15, 2023, in the Korean Intellectual Property Office, the disclosure of which is incorporated by reference herein in its entirety.

### BACKGROUND

### 1. Field

The disclosure relates to a gravity compensation control device and an ultrasonic diagnostic device including the same.

### 2. Description of the Related Art

Gravity compensation control devices are classified into an electric type and a manual type. Electric-type gravity compensation control devices use motors, and manual-type gravity compensation control devices generally use gas springs.

A gravity compensation control device used in an ultrasonic diagnostic device is a structure for compensating for the weight of a control panel or the like, and uses one or more gas springs and adjusts the weight compensation range according to the number of gas springs. However, there is an issue with using gas springs in that the pressure of the gas springs cannot be adjusted after installation, and thus, the gas springs cannot compensate for weights in response to weight changes, for example, when it is necessary to change the pressure as the weight of a control panel or the like changes after installation or the lift angle increases.

In addition, some gas springs (e.g., lockable gas springs) have structural limitations on gas pressure for internal valve control, and thus, when a compensation force greater than the gas pressure is required, it is required to increase the number of gas springs, or a separate additional compensation device is required. However, in the case of a structure using a plurality of gas springs, there are issues such as structural complexity due to an increase in the compressive force of the gas springs and an increase in the pressure for opening and closing valves.

In addition, the control ability of the control panel is inevitably affected by changes in the weight of the control panel, pressure deviation of the gas springs, pressure leakage, temperature deviation, and the like, making it difficult to use.

As described above, the existing gravity compensation control device with a fixed compensation pressure cannot compensate for the gravity in response to gravity, an external force, environmental changes, and the like, and there is also an issue that the control ability of the control panel varies depending on the user.

In recent years, modular gravity compensation control devices have been developed, which leads to replacement of gas springs or addition of separate compensation devices. However, replacing gas springs or adding separate compensation devices requires reliability and durability testing, and adaptation to changes in operating force, which may degrade usability. Therefore, it is necessary to develop technology that allows additional compensation control without replacing gas springs.

### SUMMARY

Provided are a gravity compensation control device capable of enabling additional compensation control without replacing a gas spring when an additional compensation force is required in a gravity compensation control structure, and an ultrasonic diagnostic device including the gravity compensation control device.

Additional aspects will be set forth in part in the description which follows and, in part, will be apparent from the description, or may be learned by practice of the presented embodiments of the disclosure.

In accordance with an aspect of the disclosure, a gravity compensation control device includes a first mounting unit, a rotatable link, one end of which is connected to a compensation shaft arranged in the first mounting unit and another end of which is vertically movable by a weight applied thereto, wherein a portion between the ends is supported by a fixed shaft 20 arranged in the first mounting unit, such that the link is rotatable about the fixed shaft, and a weight compensation device, wherein the weight compensation device includes a bearing unit including a bearing shaft arranged in the first mounting unit, and a bearing coupled to the bearing shaft, an auxiliary link, one end of which is connected to the compensation shaft and the other end of which is connected to one side of the bearing unit, and an elastic member coupled to another side of the bearing unit and arranged in a lengthwise direction of the auxiliary link.

In detail, the bearing unit may be capable of at least one of rotating and moving.

In detail, the bearing unit may be capable of at least one of rotating and moving along a guideline arranged in the first mounting unit.

In detail, a nut is coupled to one end of the elastic member.

In detail, the gravity compensation control device may further include a guide link at one end of the elastic member.

In detail, the weight compensation device may include a plurality of weight compensation devices.

In detail, the gravity compensation control device may further include a connection unit connecting the plurality of weight compensation devices to each other.

In detail, the respective elastic members arranged in the plurality of weight compensation devices may have different elastic forces.

In detail, the compensation shaft may be rotatable.

In detail, the gravity compensation control device may further includes a first mounting unit having a first shaft and a second shaft arranged therein, and a second mounting unit that is vertically movable and has a third shaft and a fourth shaft arranged therein, and the rotatable link may be at least one of a first link connected to the first shaft and the third shaft, a second link connected to the second shaft and the fourth shaft and arranged below the first link, a third link connected to the first shaft and the second shaft, and a fourth link connected to the third shaft and the fourth shaft.

In accordance with another aspect of the disclosure, an ultrasonic diagnostic device including the gravity compensation control device further includes a main body connected to the first mounting unit, and a control panel connected to a connection frame to be movable up, down, left, right, or diagonally with respect to the main body, wherein the connection frame includes the gravity compensation control device.

In accordance with another aspect of the disclosure, a gravity compensation control device includes a first mounting unit, a second mounting unit that is vertically movable, a main compensation device including a main elastic member, and a weight compensation device, wherein the weight compensation device includes a bearing unit including a bearing shaft arranged in the first mounting unit, and a bearing coupled to the bearing shaft, an auxiliary link, one end of which is connected to a rotatable compensation shaft arranged in the first mounting unit and another end of which is connected to one side of the bearing unit, and an elastic member coupled to another side of the bearing unit and arranged along a lengthwise direction of the auxiliary link.

In detail, the bearing unit may be capable of at least one of rotating and moving along a guideline arranged in the first mounting unit.

In detail, a nut is coupled to one end of the elastic member.

In detail, the gravity compensation control device may further include a guide link at one end of the elastic member.

In detail, the weight compensation device may include a plurality of weight compensation devices.

In detail, the gravity compensation control device may further include a connection unit connecting the plurality of weight compensation devices to each other.

In detail, the respective elastic members arranged in the plurality of weight compensation devices may have different elastic forces.

In detail, the gravity compensation control device may further include a first mounting unit having a first shaft and a second shaft arranged therein, and a second mounting unit having a third shaft and a fourth shaft arranged therein, wherein the main compensation device may include a first link connected to the first shaft and the third shaft, a second link connected to the second shaft and the fourth shaft and arranged below the first link, and a gas spring connected to the third shaft and the second shaft, and the first link may extend from the third shaft toward the first shaft such that one end of the first link is connected to the compensation shaft.

In detail, the gravity compensation control device may further include a third link connected to the first shaft and the second shaft, and a fourth link connected to the third shaft and the fourth shaft.

In accordance with another aspect of the disclosure, an ultrasonic diagnostic device includes the gravity compensation control device, and further includes a main body connected to the first mounting unit, a control panel connected to the second mounting unit, and a connection frame connecting the control panel to the main body to be movable up, down, left, right, or diagonally with respect to the main body, wherein the connection frame includes the gravity compensation control device.

### BRIEF DESCRIPTION OF THE DRAWINGS

The above and other aspects, features, and advantages of certain embodiments of the disclosure will be more apparent from the following description taken in conjunction with the accompanying drawings, in which:
FIG. 1 is a block diagram illustrating a configuration of an ultrasonic diagnostic device according to an embodiment;
FIG. 2 is a block diagram illustrating a configuration of an ultrasonic diagnostic device according to an embodiment;
FIG. 3 is a block diagram illustrating a configuration of an ultrasonic diagnostic device according to an embodiment;
(a) to (c) of FIG. 4 are perspective views of ultrasonic diagnostic devices according to at least one embodiment;
(a) to (c) of FIG. 5 are perspective views of ultrasonic diagnostic devices according to at least one embodiment;
FIG. 6 is a diagram for describing an ultrasonic diagnostic device according to an embodiment;
FIG. 7 is a diagram for describing a gravity compensation control device according to an embodiment;
FIG. 8 is a diagram for describing a gravity compensation control device according to an embodiment;
FIG. 9 is a diagram for describing a gravity compensation control device according to another embodiment;
FIG. 10 is a diagram for describing a gravity compensation control device according to another embodiment;
FIG. 11 is a diagram for describing an operation of a gravity compensation control device according to an embodiment;
FIG. 12 is a diagram for describing an operation of a gravity compensation control device according to an embodiment;
FIG. 13 is a diagram for describing a gravity compensation control device according to another embodiment; and
FIG. 14 is a diagram for describing a gravity compensation control device according to another embodiment.

### DETAILED DESCRIPTION

Reference will now be made in detail to embodiments, examples of which are illustrated in the accompanying drawings, wherein like reference numerals refer to like elements throughout. In this regard, the present embodiments may have different forms and should not be construed as being limited to the descriptions set forth herein. Accordingly, the embodiments are merely described below, by referring to the figures, to explain aspects of the present description. As used herein, the term "and/or" includes any and all combinations of one or more of the associated listed items. Expressions such as "at least one of," when preceding a list of elements, modify the entire list of elements and do not modify the individual elements of the list.

In the present specification, the principle of the disclosure is described and embodiments are provided in such a manner that the scope of the disclosure becomes apparent and the disclosure may be carried out by those of skill in the art to which the disclosure pertains. The disclosed embodiments may be implemented in various forms.

Throughout the present specification, when a part is referred to as being "connected" to another part, it may mean that the part is directly or indirectly connected to the other part, and such indirect connection includes connection through a wireless communication network.

In addition, terms used herein are for describing the embodiments and are not intended to limit the scope of the disclosure. Singular forms are intended to include plural forms as well, unless the context clearly indicates otherwise. In the present specification, the terms such as "comprise", "include", or "have" are used to designate a presence of characteristic, number, step, operation, element, component, or a combination thereof, and not to preclude a presence or a possibility of adding one or more of other characteristics, numbers, stages, operations, elements, components or a combination thereof.

In addition, although terms such as "first" or "second" may be used herein to describe various components, these components are not be limited by these terms, and these terms are used only to distinguish one component from another component. For example, a first component may be referred to as a second component, and a second component may be referred to as a first component in a similar manner, without departing from the scope of the disclosure.

In addition, terms such as "...er (or)", "... unit", "... block", "... member", or "... module" may refer to a unit that performs at least one function or operation. For example, these terms may refer to at least one hardware unit such as a field-programmable gate array (FPGA) or application-specific integrated circuit (ASIC), at least one software unit stored in a memory, or at least one process performed by a processor.

Reference numerals assigned to respective operations are to identify the operations, and do not indicate the order of the operations, and each operation may be performed differently from a described order unless the context clearly indicates a particular order.

In addition, the term "image" used herein may include medical images obtained by medical imaging devices such as magnetic resonance imaging (MRI) devices, computed tomography (CT) devices, ultrasonic imaging devices, or X-ray imaging devices, and medical images of other modalities in addition to ultrasound images may also be provided or controlled.

In addition, the term 'object' used herein refers to a target to be photographed, and may include a person, an animal, or part thereof. For example, an object may include part of a human body (e.g., an organ), a phantom, and the like.

Throughout the present specification, the term "ultrasound image" refers to an image of an object that is obtained by processing ultrasonic signals emitted toward and reflected from an object.

Hereinafter, embodiments will be described in detail with reference to the accompanying drawings.

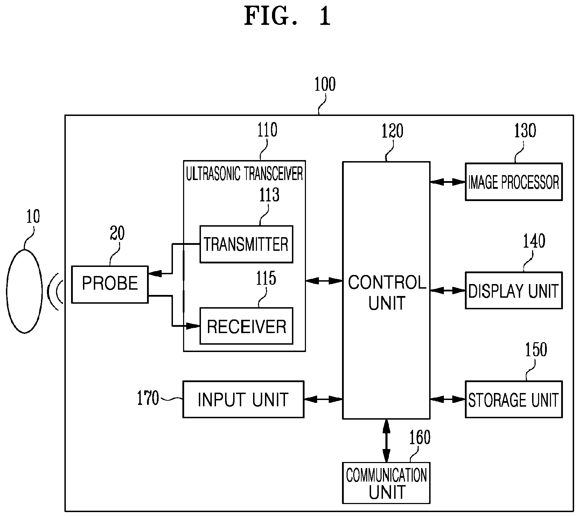

FIG. 1 is a block diagram illustrating a configuration of an ultrasonic diagnostic device 100 according to an embodiment.

The ultrasonic diagnostic device 100 according to an embodiment may include a probe 20, an ultrasonic transceiver 110, a control unit 120, an image processor 130, a display unit 140, a storage unit 150, and a communication unit 160, and an input unit 170.

The ultrasonic diagnostic device 100 may be implemented not only as a cart type but also as a portable type. Examples of portable ultrasonic diagnostic devices may include, but are not limited to, a smart phone, a laptop computer, a personal digital assistant (PDA), and a tablet personal computer (PC) that include a probe and an application.

The probe 20 may include a plurality of transducers. The plurality of transducers may transmit ultrasonic signals to an object 10 according to transmission signals applied from a transmitter 113. The plurality of transducers may receive the ultrasonic signals reflected from the object 10 to generate reception signals. In addition, the probe 20 may be implemented integrally with the ultrasonic diagnostic device 100, or may be implemented as a separate unit connected to the ultrasonic diagnostic device 100 in a wired or wireless manner. In addition, the ultrasonic diagnostic device 100 may include one or more probes 20 according to an implementation.

The control unit 120 controls the transmitter 113 to generate transmission signals to be applied to the plurality of transducers included in the probe 20, respectively, considering the positions and focal points of the plurality of transducers.

The control unit 120 controls a receiver 115 to generate ultrasound data by converting the reception signals received from the probe 20, from analog signals to digital signals, and summing the reception signals converted to digital signals, considering the positions and focal points of the plurality of transducers.

The image processor 130 generates an ultrasound image by using the ultrasound data generated by the receiver 115.

Meanwhile, an ultrasound image may be generated not only as a grayscale ultrasound image obtained by scanning an object in an amplitude mode (A-mode), a brightness mode (B-mode), and a motion mode (M-mode), but also as a Doppler image of a motion of the object.

The A-mode is the most basic form of ultrasound image display, and is for displaying the intensity of a reflected sound as an amplitude on a time (distance) axis. As the intensity of the reflected sound increases, the amplitude also increases, and vice versa. Thus, the A-mode is advantageous for distance measurement, but a resulting image changes even when the direction of the probe is slightly changed, and thus it is rarely used today.

The M-mode is a modification of the A-mode, and is for displaying the distance to a moving reflector as a temporal variation. The M-mode is for designating a region of interest (ROI) in a two-dimensional (2D) image as an M line and displaying changes in the ROI over time, and is primarily used for observation of cardiac valves. The M-mode may also be used for recording fetal heart sounds, however, the Doppler method is increasingly replacing the M-mode.

The B-mode refers to a method of displaying a reflected sound as the brightness of a dot, and is used in most ultrasound diagnostic devices today. The brightness of each dot is proportional to the amplitude of a reflected signal. Recently, it provides 256 brightness levels or more, and also displays a motion of an organ in real time. The B-mode is also referred to as 2D mode, and is for displaying a cross-sectional image of an object in real time on a screen image in black and white shades. It is most frequently used.

In addition, a Doppler mode is generally for measuring blood flow by detecting the flow of red blood cells in a blood vessel. It uses the principle that the wavelength becomes shorter when the red blood cells approach a prove and longer when they move away from the prove. Examples of Doppler modes include color Doppler, pulse wave Doppler (PW), and continuous wave Doppler (CW), depending on a method of displaying blood flow. Doppler images may include blood flow Doppler images (also referred to as color Doppler images) showing blood flow, tissue Doppler images showing a motion of tissue, and spectral Doppler images showing the moving speed of an object as a waveform.

In addition, as combination modes, there are a mode for displaying different modes based on 2D by simultaneously applying two or three modes to one image, and a three-dimensional (3D) mode for displaying a three-dimensional image.

In B-mode processing, B-mode components may be extracted from ultrasound data and processed. In an image generation process, an ultrasound image expressing the intensity of a signal as brightness based on the B-mode components extracted in the B-mode processing may be generated. In Doppler processing, Doppler components may be extracted from ultrasound data. In an image generation process, a Doppler image expressing a motion of the object 10 in color or waveform may be generated based on the extracted Doppler components.

In the image generation process, a 2D ultrasound image or a 3D image of the object may be generated, and an elastic image expressing the degree of deformation of the object 10 according to pressure may also be generated. Furthermore, various pieces of additional information may be expressed as text or graphics on the ultrasound image. Meanwhile, the generated ultrasound image may be stored in a memory.

In a process of measuring an object in an ultrasound image, a measurement tool for measuring the object may be determined, and any one of a plurality of measurement tools may be selected based on a user input.

For example, a measurement tool selection menu may be provided for selecting any one of the plurality of measurement tools, and the measurement tool selection menu may be displayed along with the ultrasound image on one screen. In addition, the measurement tool selection menu may be displayed on a separate screen from a touch screen on which the ultrasound image is displayed.

In addition, any one of the plurality of measurement tools may be determined based on a user input for selecting one of the plurality of measurement items to be measured. Measurement items may include, but are not limited to, length, area, or angle.

Based on receiving a user input for selecting nay one of the measurement items, a predetermined measurement tool corresponding to the selected measurement item may be determined.

The display unit 140 may display the generated ultrasound image and various pieces of information processed by the ultrasonic diagnostic device 100. The ultrasonic diagnostic device 100 may include one or more display units 140 according to an implementation. In addition, the display unit 140 may be implemented as a touch screen in combination with a touch panel.

The control unit 120 may control the overall operation of the ultrasonic diagnostic device 100 and signal flow between internal components of the ultrasonic diagnostic device 100. The control unit 120 may include a memory storing programs or data for performing functions of the ultrasonic diagnostic device 100, and a processor configured to process the programs or data. In addition, the control unit 120 may receive a control signal from the input unit 170 or an external device to control an operation of the ultrasonic diagnostic device 100.

The ultrasonic diagnostic device 100 may include the communication unit 160 and may be connected to an external device (e.g., a server, a medical device, or a portable device (e.g., a smart phone, a tablet PC, or a wearable device)) through the communication unit 160.

The communication unit 160 may include one or more components configured to enable communication with the external device, and may include, for example, at least one of a short-range communication module, a wired communication module, and a wireless communication module.

The communication unit 160 may receive a control signal and data from the external device, and transmit the received control signal to the control unit 120, such that the control unit 120 controls the ultrasonic diagnostic device 100 according to the received control signal.

Alternatively, the control unit 120 may transmit a control signal to the external device through the communication unit 160, to control the external device according to the control signal from the control unit 120.

For example, the external device may process data of the external device according to a control signal received from the control unit 120 through a communication unit.

A program (e.g., artificial intelligence) for controlling the ultrasonic diagnostic device 100 may be installed in the external device, and the program may include instructions for performing some or all of operations of the control unit 120.

The program may be installed in the external device in advance, or a user of the external device may download and install the program from a server for providing applications. The server for providing applications may include a recording medium storing the program.

In addition, in a system including a server and a client device, the program may include a storage medium of the server or a storage medium of the client device. Alternatively, in a case in which a third device (e.g., a smart phone, a tablet PC, or a wearable device) is communicatively connected to the server or client device, a program product may include a storage medium of the third device. Alternatively, the computer may include a software program that is transmitted from the server to the client device or the third device or transmitted from the third device to the client device.

In this case, any one of the server, the client device, and the third device may execute the program to perform the method according to the disclosed embodiments. Alternatively, two or more of the server, the client device, and the third device may execute the program to execute the method according to the disclosed embodiments in a distributed manner.

For example, the server (e.g., a cloud server or an artificial intelligence server) may execute the program stored in the server to control the client device communicatively connected to the server to perform the method according to the disclosed embodiments.

The storage unit 150 may store various pieces of data or programs for operating and controlling the ultrasonic diagnostic device 100, input/output ultrasound data, obtained ultrasound images, and the like.

The input unit 170 may receive a user input for controlling the ultrasonic diagnostic device 100. For example, the user input may include, but is not limited to, an input of manipulating a button, a keypad, a mouse, a trackball, a jog switch, a knob, or the like, an input of touching a touch pad or a touch screen, a voice input, a motion input, a biometric information input (e.g., iris recognition or fingerprint recognition), and the like.

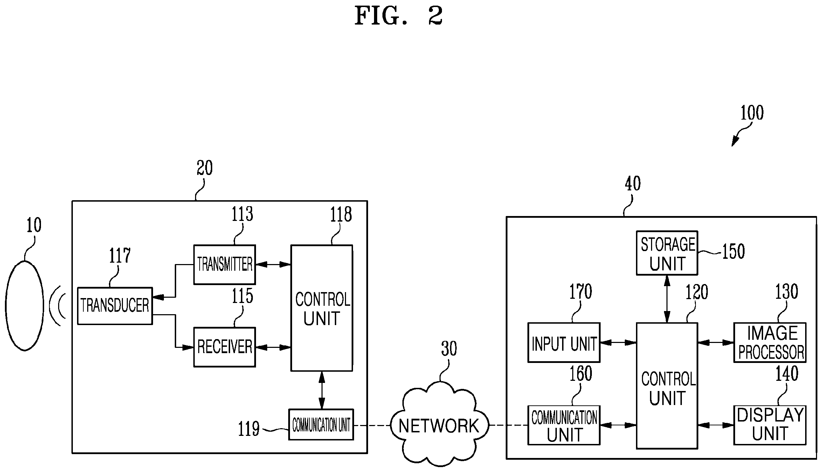

FIG. 2 is a block diagram illustrating a configuration of the ultrasonic diagnostic device 100 according to an embodiment.

Referring to FIG. 2, the ultrasonic diagnostic device 100 may include a wireless probe 20 and an ultrasound system 40.

The wireless probe 20 may include the transmitter 113, a transducer 117, the receiver 115, a control unit 118, and a communication unit 119. Although FIG. 2 illustrates that the wireless probe 20 includes both the transmitter 113 and the receiver 115, the wireless probe 20 may include only some of the components of the transmitter 113 and the receiver 115 and some of the components of the transmitter 113 and the receiver 115 may be included in the ultrasound system 40, according to an implementation. Alternatively, the wireless probe 20 may further include the image processor 130.

The transducer 117 may include a plurality of transducers. The plurality of transducers may transmit ultrasonic signals to the object 10 according to transmission signals applied from the transmitter 113. The plurality of transducers may receive the ultrasonic signals reflected from the object 10 to generate reception signals.

The control unit 118 controls the transmitter 113 to generate transmission signals to be applied to the plurality of transducers, respectively, considering the positions and focal points of the plurality of transducers.

The control unit 118 controls the receiver 115 to generate ultrasound data by converting the reception signals received from the transducer 117, from analog signals to digital signals, and summing the reception signals converted to digital signals, considering the positions and focal points of the plurality of transducers. Alternatively, in a case in which the wireless probe 20 includes the image processor 130, an ultrasound image may be generated by using the generated ultrasound data.

The communication unit 119 may wirelessly transmit the generated ultrasound data or ultrasound image to the ultrasound system 40 through a wireless network. Alternatively, the communication unit 119 may receive a control signal and data from the ultrasound system 40.

In addition, the ultrasonic diagnostic device 100 may include one or more wireless probes 20 according to an implementation.

The ultrasound system 40 may receive the ultrasound data or ultrasound image from the wireless probe 20. The ultrasound system 40 may include the control unit 120, the image processor 130, the display unit 140, the storage unit 150, the communication unit 160, and the input unit 170.

The image processor 130 generates an ultrasound image by using the ultrasound data received from the wireless probe 20.

The display unit 140 may display the ultrasound image received from the wireless probe 20, the ultrasound image generated by the ultrasound system 40, and various pieces of information processed by the ultrasonic diagnostic device 100. The ultrasonic diagnostic device 100 may include one or more display units 140 according to an implementation. In addition, the display unit 140 may be implemented as a touch screen in combination with a touch panel.

The control unit 120 may control the overall operation of the ultrasonic diagnostic device 100 and signal flow between internal components of the ultrasonic diagnostic device 100. The control unit 120 may include a memory storing programs or data for performing functions of the ultrasonic diagnostic device 100, and a processor configured to process the programs or data. In addition, the control unit 120 may receive a control signal from the input unit 170 or an external device to control an operation of the ultrasonic diagnostic device 100.

The ultrasound system 40 may include the communication unit 160 and may be connected to an external device (e.g., a server, a medical device, or a portable device (e.g., a smart phone, a tablet PC, or a wearable device)) through the communication unit 160.

The communication unit 160 may include one or more components configured to enable communication with the external device, and may include, for example, at least one of a short-range communication module, a wired communication module, and a wireless communication module.

The communication unit 160 may receive a control signal and data from the external device, and transmit the received control signal to the control unit 120, such that the control unit 120 controls the ultrasonic diagnostic device 100 according to the received control signal.

Alternatively, the control unit 120 may transmit a control signal to the external device through the communication unit 160, to control the external device according to the control signal from the control unit 120.

For example, the external device may process data of the external device according to a control signal received from the control unit 120 through a communication unit.

A program (e.g., artificial intelligence) for controlling the ultrasonic diagnostic device 100 may be installed in the external device, and the program may include instructions for performing some or all of operations of the control unit 120.

The program may be installed in the external device in advance, or a user of the external device may download and install the program from a server for providing applications. The server for providing applications may include a recording medium storing the program.

In addition, in a system including a server and a client device, the program may include a storage medium of the server or a storage medium of the client device. Alternatively, in a case in which a third device (e.g., a smart phone, a tablet PC, or a wearable device) is communicatively connected to the server or client device, a program product may include a storage medium of the third device. Alternatively, the computer may include a software program that is transmitted from the server to the client device or the third device or transmitted from the third device to the client device.

In this case, any one of the server, the client device, and the third device may execute the program to perform the method according to the disclosed embodiments. Alternatively, the client device may perform the method according to the disclosed embodiments via the server.

Alternatively, two or more of the server, the client device, and the third device may execute the program to execute the method according to the disclosed embodiments in a distributed manner.

For example, the server (e.g., a cloud server or an artificial intelligence server) may execute the program stored in the server to control the client device communicatively connected to the server to perform the method according to the disclosed embodiments.

The storage unit 150 may store various pieces of data or programs for operating and controlling the ultrasonic diagnostic device 100, input/output ultrasound data, ultrasound images, and the like.

The input unit 170 receives a user input for controlling the ultrasonic diagnostic device 100. For example, the user input may include, but is not limited to, an input of manipulating a button, a keypad, a mouse, a trackball, a jog switch, a knob, or the like, an input of touching a touch pad or a touch screen, a voice input, a motion input, a biometric information input (e.g., iris recognition or fingerprint recognition), and the like.

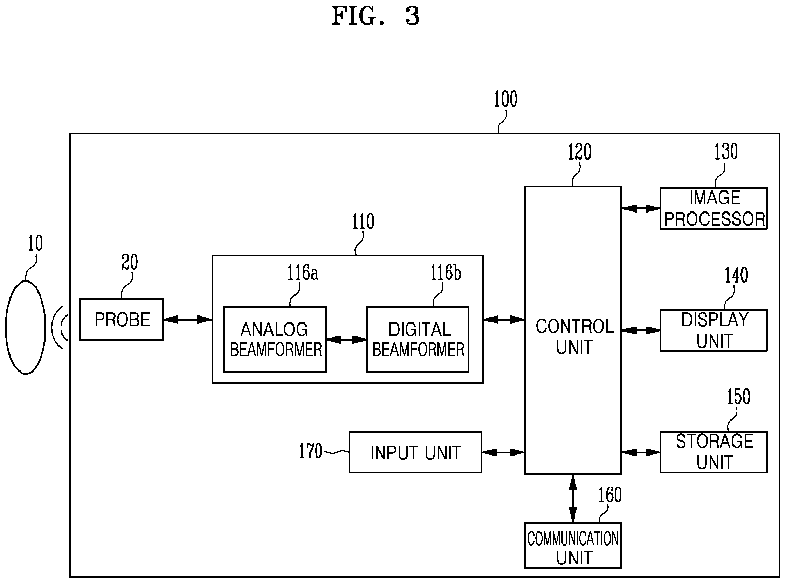

FIG. 3 is a block diagram illustrating a configuration of the ultrasonic diagnostic device 100 according to an embodiment.

Referring to FIG. 3, the ultrasonic diagnostic device 100 may include the probe 20, the ultrasonic transceiver 110, the control unit 120, the image processor 130, the display unit 140, the input unit 170, the storage unit 150, and the communication unit 160.

The probe 20 according to an embodiment may include a plurality of transducers. The plurality of transducers may be arranged in two dimensions to form a two-dimensional transducer array.

For example, the two-dimensional transducer array may include a plurality of sub-arrays including a plurality of transducers arranged in a first direction, wherein the plurality of sub-arrays are arranged in a second direction different from the first direction.

In addition, the ultrasonic transceiver 110 may include an analog beamformer 116a and a digital beamformer 116b. Although FIG. 3 illustrates that the ultrasonic transceiver 110 and the probe 20 are separate components, the probe 20 according to an embodiment may include some or all of the components of the ultrasonic transceiver 110 according to an implementation. For example, the probe 20 may include any one or both of the analog beamformer 116a and the digital beamformer 116b.

The control unit 120 may calculate a time delay value for digital beamforming for each of the plurality of sub-arrays included in the two-dimensional transducer array. In addition, the control unit 120 may calculate a time delay value for analog beamforming for each transducer included in any one of the plurality of sub-arrays.

The control unit 120 may control the analog beamformer 116a and the digital beamformer 116b to generate a transmission signal to be applied to each of the plurality of transducers, according to the time delay values for analog beamforming and the time delay values for digital beamforming.

In addition, the control unit 120 may control the analog beamformer 116a to sum signals received from the plurality of transducers for each sub-array, according to the time delay values for analog beamforming. In addition, the control unit 120 may control the ultrasonic transceiver 110 to convert a result of summing the signals for each sub-array, from analog signals to digital signals. In addition, the control unit 120 may control the digital beamformer 116b to generate ultrasound data by summing the signals converted to digital signals, according to the time delay values for digital beamforming.

The image processor 130 generates an ultrasound image by using the generated ultrasound data.

The display unit 140 may display the generated ultrasound image and various pieces of information processed by the ultrasonic diagnostic device 100. The ultrasonic diagnostic device 100 may include one or more display units 140 according to an implementation. In addition, the display unit 140 may be implemented as a touch screen in combination with a touch panel.

The control unit 120 may control the overall operation of the ultrasonic diagnostic device 100 and signal flow between internal components of the ultrasonic diagnostic device 100. The control unit 120 may include a memory storing programs or data for performing functions of the ultrasonic diagnostic device 100, and a processor configured to process the programs or data. In addition, the control unit 120 may receive a control signal from the input unit 170 or an external device to control an operation of the ultrasonic diagnostic device 100.

The ultrasonic diagnostic device 100 may include the communication unit 160 and may be connected to an external device (e.g., a server, a medical device, or a portable device (e.g., a smart phone, a tablet PC, or a wearable device)) through the communication unit 160.

The communication unit 160 may include one or more components configured to enable communication with the external device, and may include, for example, at least one of a short-range communication module, a wired communication module, and a wireless communication module.

The communication unit 160 may receive a control signal and data from the external device, and transmit the received control signal to the control unit 120, such that the control unit 120 controls the ultrasonic diagnostic device 100 according to the received control signal.

Alternatively, the control unit 120 may transmit a control signal to the external device through the communication unit 160, to control the external device according to the control signal from the control unit 120.

For example, the external device may process data of the external device according to a control signal received from the control unit 120 through a communication unit.

A program (e.g., artificial intelligence) for controlling the ultrasonic diagnostic device 100 may be installed in the external device, and the program may include instructions for performing some or all of operations of the control unit 120.

The program may be installed in the external device in advance, or a user of the external device may download and install the program from a server for providing applications. The server for providing applications may include a recording medium storing the program.

In addition, in a system including a server and a client device, the program may include a storage medium of the server or a storage medium of the client device. Alternatively, in a case in which a third device (e.g., a smart phone, a tablet PC, or a wearable device) is communicatively connected to the server or client device, a program product may include a storage medium of the third device. Alternatively, the computer may include a software program that is transmitted from the server to the client device or the third device or transmitted from the third device to the client device.

In this case, any one of the server, the client device, and the third device may execute the program to perform the method according to the disclosed embodiments. Alternatively, two or more of the server, the client device, and the third device may execute the program to execute the method according to the disclosed embodiments in a distributed manner.

For example, the server (e.g., a cloud server or an artificial intelligence server) may execute the program stored in the server to control the client device communicatively connected to the server to perform the method according to the disclosed embodiments.

The storage unit 150 may store various pieces of data or programs for operating and controlling the ultrasonic diagnostic device 100, input/output ultrasound data, ultrasound images, and the like.

The input unit 170 may receive a user input for controlling the ultrasonic diagnostic device 100. For example, the user input may include, but is not limited to, an input of manipulating a button, a keypad, a mouse, a trackball, a jog switch, a knob, or the like, an input of touching a touch pad or a touch screen, a voice input, a motion input, a biometric information input (e.g., iris recognition or fingerprint recognition), and the like.

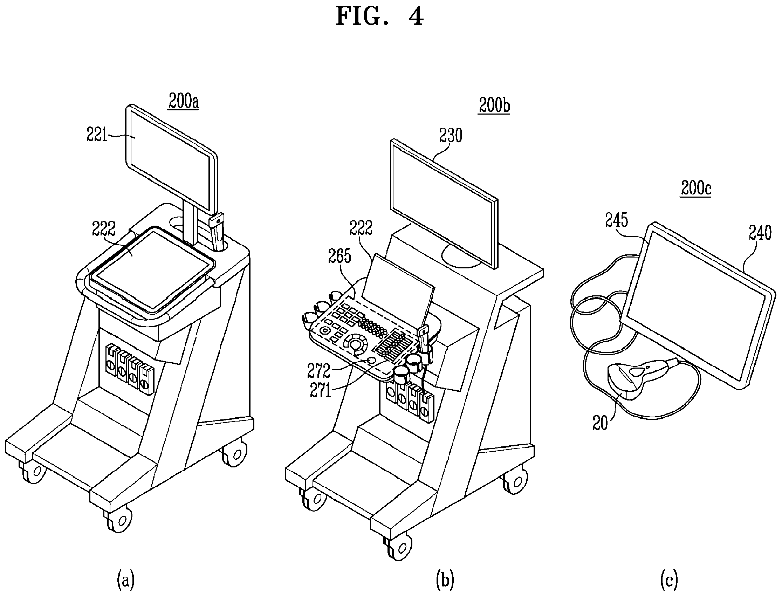

(a) to (c) of FIG. 4 are perspective views of ultrasonic diagnostic devices 200a, 200b, and 200c according to at least one embodiment.

Referring to (a) and (b) of FIG. 4, the ultrasonic diagnostic devices 200a and 200b may include a main display unit 221 and a sub-display unit 222. Any one of the main display unit 221 and the sub-display unit 222 may be implemented as a touch screen. The main display unit 221 and the sub-display unit 222 may display ultrasound images or various pieces of information processed by the ultrasonic diagnostic device 200a or 200b. In addition, the main display unit 221 and the sub-display unit 222 may be implemented as touch screens and provide a graphical user interface (GUI), such that data for controlling the ultrasonic diagnostic device 200a or 200b may be input from a user. For example, the main display unit 221 may display an ultrasound image, and the sub-display unit 222 may display a control panel for controlling the display of the ultrasound image, in a GUI form. The sub-display unit 222 may receive data for controlling the display of the image, through the control panel displayed in the GUI form. The ultrasonic diagnostic device 200a or 200b may control the display of the ultrasound image on the main display unit 221 by using the input control data.

Referring to (b) of FIG. 4, the ultrasonic diagnostic device 200b may further include a control panel 265 in addition to the main display unit 221 and the sub-display unit 222. The control panel 265 may include a button, a trackball, a jog switch, a knob, and the like, and may receive an input of data for controlling the ultrasonic diagnostic device 200b, from the user. For example, the control panel 265 may include a time gain compensation (TGC) button 271, a Freeze button 272, and the like. The TGC button 271 is for setting a TGC value for each depth of an ultrasound image. In addition, based on detecting an input of the Freeze button 272 while scanning an ultrasound image, the ultrasonic diagnostic device 200b may maintain a state in which a frame image at a time point of the detecting is displayed.

Meanwhile, the button, trackball, jog switch, knob, and the like included in the control panel 265 may be provided as a GUI on the main display unit 221 or the sub-display unit 222.

Referring to (c) of FIG. 4, the ultrasonic diagnostic device 200c may be implemented as a portable device. Examples of portable ultrasonic diagnostic devices 200c may include, but are not limited to, a smart phone, a laptop computer, a PDA, and a tablet PC that include a probe and an application.

The ultrasonic diagnostic device 200c may include the probe 20 and a main body 240, and the probe 20 may be connected to one side of the main body 240 in a wired or wireless manner. The main body 240 may include a touch screen 245. The touch screen 245 may display an ultrasound image, various pieces of information processed by the ultrasonic diagnostic device, a GUI, and the like.

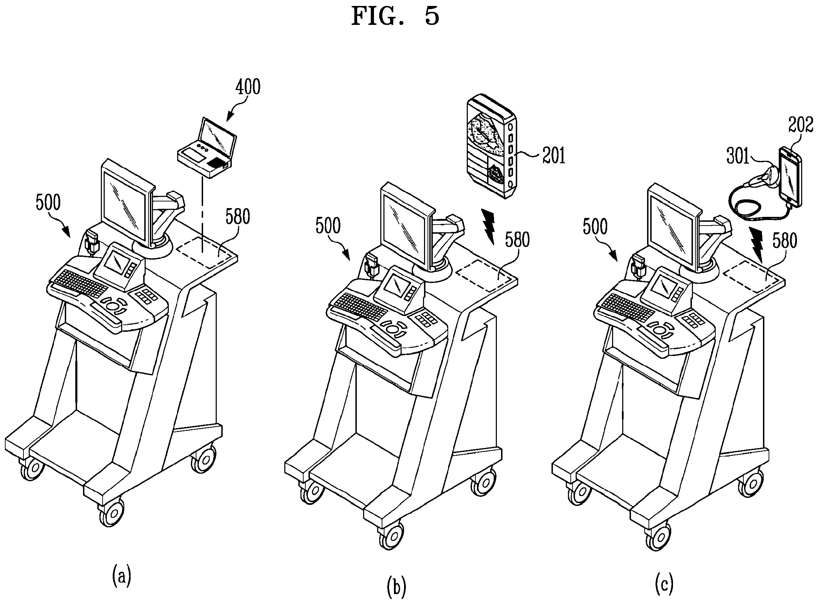

(a) to (c) of FIG. 5 are perspective views of an ultrasonic diagnostic device 500 according to at least one embodiment.

Referring to (a) of FIG. 5, the indoor ultrasonic diagnostic device 500 refers to a non-portable ultrasonic diagnostic device generally used for ultrasonic diagnosis, and is also referred to as a cart-based device. The ultrasonic diagnostic device 500 does not necessarily have to be used only indoors, but for convenience of description, will be referred to as the indoor ultrasonic diagnostic device 500.

The indoor ultrasonic diagnostic device 500 may have a portable docking unit 580 connected to a portable ultrasonic diagnostic device 400, and because other components of the indoor ultrasonic diagnostic device 500 used in the disclosure than the portable docking unit 580 are commonly used, detailed descriptions thereof will be omitted.

Unlike the portable ultrasonic diagnostic device 400, the indoor ultrasonic diagnostic device 500 has fewer constraints in terms of size, weight, and power consumption, thus may be used for diagnosis of various diseases, and may be developed for high performance. When the portable ultrasonic diagnostic device 400 is mounted on the indoor ultrasonic diagnostic device 500, the portable ultrasonic diagnostic device 400 may be used with high performance. However, the portable ultrasonic diagnostic device 400 may be any position on the indoor ultrasonic diagnostic device 500 without limitation as long as it is convenient for the user to simultaneously use the portable ultrasonic diagnostic device 400 and the indoor ultrasonic diagnostic device 500, and is not limited to the position illustrated in (a) of FIG. 5. Furthermore, the portable ultrasonic diagnostic device 400 may be connected to the indoor ultrasonic diagnostic device 500 through a wire or by being integrated with the indoor ultrasonic diagnostic device 500.

Referring to (a) and (b) of FIG. 5, the portable ultrasonic diagnostic device 400 of (a) may correspond to a portable ultrasonic diagnostic device 201 of (b).

The portable ultrasonic diagnostic device 400 may be formed integrally with a probe (not shown) including a plurality of transducer elements. In detail, the portable ultrasonic diagnostic device 400 refers to a device that is connected to the indoor ultrasonic diagnostic device 500 by using a wireless or wired communication method (including a Universal Serial Bus (USB) communication method), to provide an ultrasound image to the user by using received ultrasound image data. For example, the portable ultrasonic diagnostic device 400 may be a smart device capable of downloading and installing an application, such as a smart phone.

In detail, the portable ultrasonic diagnostic device 400 may be a device that is connected to the indoor ultrasonic diagnostic device 500 by using a wired or wireless communication method, to provide an ultrasound image to the user by using received ultrasound image data.

For example, the wireless communication method may include at least one short-range data communication method, such as a 60-GHz (mmWave) wireless local area network (WLAN). The wireless communication method may be WLAN (e.g., Wi-Fi), Bluetooth, ZigBee, Wi-Fi Direct (WFD), Infrared Data Association (IrDA), Bluetooth Low Energy (BLE), near-field communication (NFC), wireless broadband (WiBro), Shared Wireless Access Protocol (SWAP) for Worldwide Interoperability for Microwave Access (WiMAX), Wireless Gigabit Alliance (WiGig), or radio-frequency (RF) communication.

(b) of FIG. 5 illustrates an example of an ultrasonic diagnostic system in which the portable ultrasonic diagnostic device 201 is connected to the cart-based ultrasonic diagnostic device 500.

The cart-based ultrasonic diagnostic device 500 may be connected to the portable ultrasonic diagnostic device 201 by using the wireless communication method described above. In detail, the portable ultrasonic diagnostic device 201 may include at least one wireless communication module (not shown) for performing at least one of the wireless communication methods described above. Furthermore, the portable docking unit 580 of the cart-based ultrasonic diagnostic device 500 may include at least one wireless communication module (not shown) for performing wireless communication with the portable ultrasonic diagnostic device 201.

In this case, the wireless communication module in the cart-based ultrasonic diagnostic device 500 may be a module for performing communication according to at least one of the wireless communication methods described above.

(c) of FIG. 5 illustrates an example of an ultrasonic diagnostic system in which a portable ultrasonic diagnostic device 202 is connected to the cart-based ultrasonic diagnostic device 500.

The portable ultrasonic diagnostic device 202 may be coupled to a probe 301 through a probe port. The portable ultrasonic diagnostic device 202 may generate an ultrasound image by using an ultrasound image corresponding to a ultrasonic signal received by the probe 301, and display the generated ultrasound image on the display unit.

The cart-based ultrasonic diagnostic device 500 may be connected to the portable ultrasonic diagnostic device 202 by using the wireless communication method described above. The connection between the cart-based ultrasonic diagnostic device 500 and the portable ultrasonic diagnostic device 202 through wireless communication corresponds to the connection between the cart-based ultrasonic diagnostic device 500 and the portable ultrasonic diagnostic device 201, and thus, a detailed description thereof will be omitted.

Hereinafter, an embodiment of an ultrasonic device that may be applied to at least one of the ultrasonic diagnostic devices described above with reference to FIGS. 1 to 3 will be described.

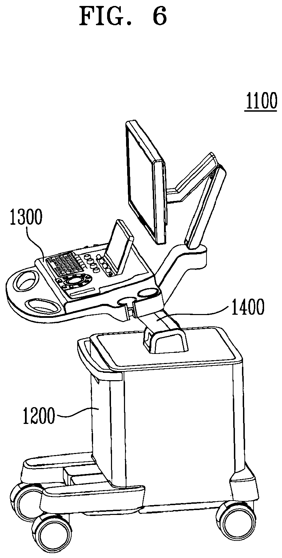

FIG. 6 is a diagram for describing an ultrasonic diagnostic device 1100 according to an embodiment.

FIG. 6 is a simplified illustration of the ultrasonic diagnostic device 1100 according to an embodiment, and the ultrasonic diagnostic device 1100 of the disclosure may include a main body 1200, a control panel 1300, and a connection frame 1400.

The main body 1200 may be movable by using a moving unit such as a wheel, or may be fixed to a particular position without mobility. The main body 1200 may be an independently distinguishable component, or may be provided on one side of other equipment so as to be difficult to be separated, but distinguishable.

The control panel 1300 may be spaced apart from the main body 1200 and connected to the main body 1200 through the connection frame 1400 to be movable up, down, left, right, or diagonally with respect to the main body 1200.

The ultrasonic diagnostic device 1100 according to an embodiment is not limited to the structure illustrated in FIG. 6, and may include any structure including the main body 1200, the control panel 1300, and the connection frame 1400 connecting them.

Although not illustrated in FIG. 6, the connection frame 1400 includes a gravity compensation control device 700 to be described below.

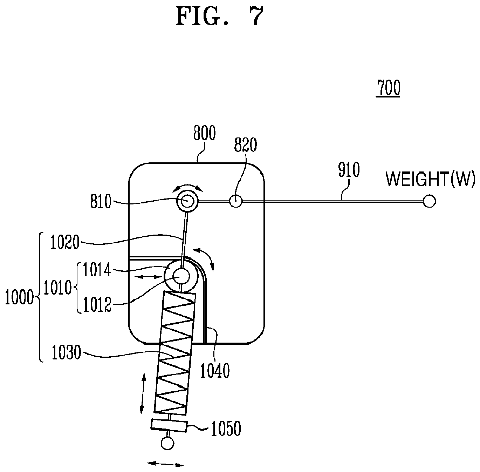

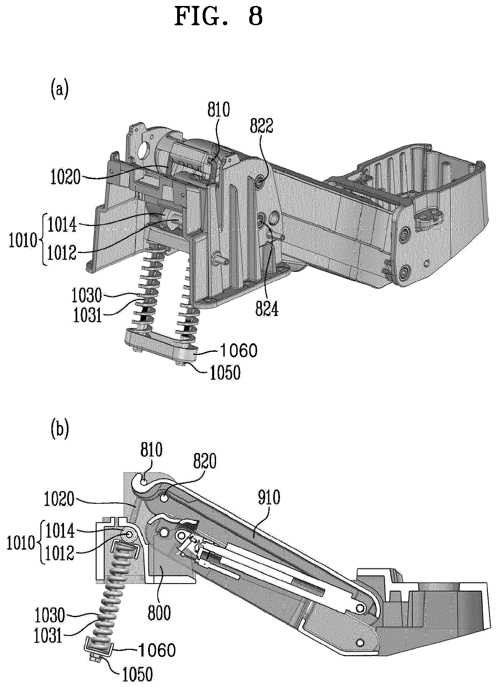

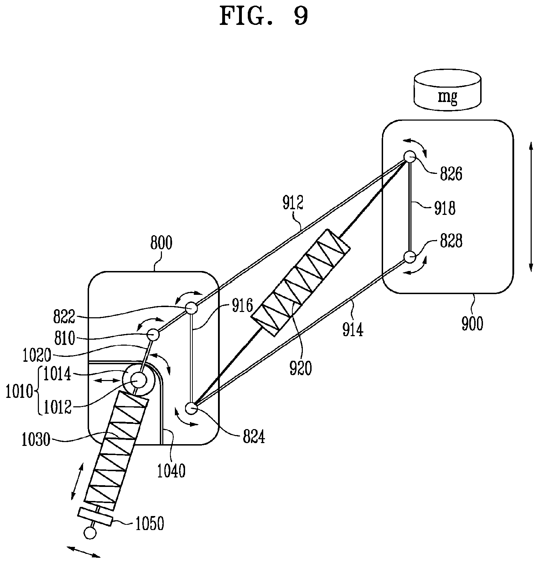

In detail, referring to FIGS. 7 and 8, according to an embodiment, the main body 1200 of the ultrasonic diagnostic device 1100 according to the disclosure may be connected to a first mounting unit 800 of the gravity compensation control device 700. In addition, referring to FIG. 9, according to another embodiment, the main body 1200 of the ultrasonic diagnostic device 1100 according to the disclosure may be connected to the first mounting unit 800, and the control panel 1300 may be connected to a second mounting unit 900.

FIG. 7 is a diagram for describing a gravity compensation control device according to an embodiment.

FIG. 8 is a diagram for describing a gravity compensation control device according to an embodiment.

As illustrated in FIGS. 7 and 8, the gravity compensation control device 700 according to an embodiment includes the first mounting unit 800, a link 910, and a weight compensation device 1000, wherein one end of the link 910 is connected to a compensation shaft 810 arranged in the first mounting unit 800, the other end of the link 910 is vertically movable by a weight applied thereto, and a portion between the ends is supported by a fixed shaft 820 arranged in the first mounting unit 800 such that the link 910 is rotatable about the fixed shaft 820.

The weight compensation device 1000 may include a bearing unit 1010 including a bearing shaft 1012 arranged in the first mounting unit 800 and a bearing 1014 coupled to the bearing shaft 1012, an auxiliary link 1020, one end of which is connected to the rotatable compensation shaft 810 and the other end of which is connected to one side of the bearing unit 1010, and an elastic member 1030 coupled to the other side of the bearing unit 1010 and arranged in the lengthwise direction of the auxiliary link 1020.

According to an embodiment, the elastic member 1030 may include rubber, a gas spring, and a spring, but is not limited thereto, and may be any member with elasticity.

In the disclosure, the compensation shaft 810 serves to generate a compensation force by causing a change in the height of the elastic member 1030, and the bearing shaft 1012 serves as the central rotation axis of the weight compensation device 1000.

As illustrated in (a) of FIG. 8, a shaft 1031 arranged to pass through the elastic member 1030 may be connected to the auxiliary link 1020 connected to the upper end of the elastic member 1030, that is, to one side of the bearing unit 1010, and the auxiliary link 1020 may be connected to the compensation shaft 810.

The gravity compensation control device 700 according to the disclosure illustrated in FIGS. 7 and 8 may be used alone without a main compensation device using a separate main elastic member, for example, a gas spring.

However, as will be described below, the gravity compensation control device 700 may be used with a main elastic member such as a gas spring 920, as a main compensation device, and in another embodiment, may also be used as a compensation structure when the output of a motor in an electric lift is insufficient.

According to an embodiment, the gravity compensation control device 700 may include the gas spring 920 connected to a third shaft 826 and a second shaft 824, and in another embodiment, may be used with various compensation structures such as coil springs or actuators.

Although not illustrated in FIGS. 7 and 8, according to an embodiment, the gravity compensation control device 700 according to the disclosure may include the first mounting unit 800 having a first shaft 822 and the second shaft 824 arranged therein, and the second mounting unit 900 vertically movable and having the third shaft 826 and a fourth shaft 828 arranged therein.

The rotatable link 910 illustrated in FIGS. 7 and 8 may be at least one of a first link 912 connected to the first shaft 822 and the third shaft 826, a second link 914 connected to the second shaft 824 and the fourth shaft 828 and arranged below the first link 912, a third link 916 connected to the first shaft 822 and the second shaft 824, and a fourth link 918 connected to the third shaft 826 and the fourth shaft 828. The fixed shaft 820 is arranged in the first mounting unit 800 and may be the first shaft 822 or the second shaft 824, and although not illustrated, the fixed shaft 820 may be the third shaft 826 or the fourth shaft 828 arranged in the second mounting unit 900.

Preferably, in the gravity compensation control device 700 according to the disclosure, the rotatable link 910 connected to the compensation shaft 810 may be any one of the first link 912 and the second link 914, and the weight compensation device 1000 according to the disclosure may be connected to the link 910 connected to the first mounting unit 800.

FIG. 9 is a diagram for describing a gravity compensation control device according to another embodiment.

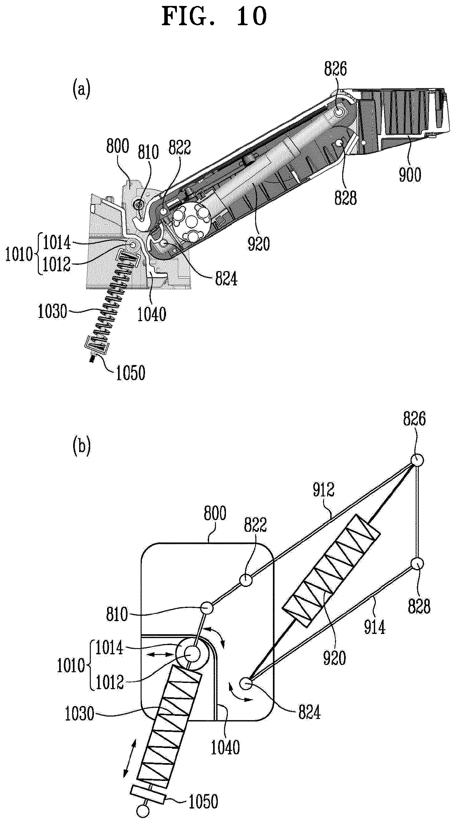

FIG. 10 is a diagram for describing a gravity compensation control device according to another embodiment.

As illustrated in FIGS. 9 and 10, the gravity compensation control device 700 according to another embodiment includes the first mounting unit 800, the second mounting unit 900 that is vertically movable, a main compensation device including a main elastic member, and the weight compensation device 1000.

The weight compensation device 1000 may include the bearing unit 1010 including the bearing shaft 1012 arranged in the first mounting unit 800 and the bearing 1014 coupled to the bearing shaft 1012, the auxiliary link 1020, one end of which is connected to the rotatable compensation shaft 810 arranged in the first mounting unit 800 and the other end of which is connected to one side of the bearing unit 1010, and the elastic member 1030 coupled to the other side of the bearing unit 1010 and arranged in the lengthwise direction of the auxiliary link 1020.

The difference between the embodiment of FIGS. 9 and 10 and the embodiment of FIGS. 7 and 8 is that the gravity compensation control device 700 illustrated in FIGS. 7 and 8 may be used alone without a separate main elastic member, for example, a gas spring, whereas the gravity compensation control device 700 illustrated in FIGS. 9 and 10 includes a main compensation device including a main elastic member, for example, the gas spring 920.

As illustrated in FIGS. 9 and 10, the gravity compensation control device 700 according to the disclosure may include the first mounting unit 800 in which the first shaft 822 and the second shaft 824 are arranged, and the second mounting unit 900 in which the third shaft 826 and the fourth shaft 828 are arranged. In addition, the main compensation device may include the first link 912 connected to the first shaft 822 and the third shaft 826, the second link 914 connected to the second shaft 824 and the fourth shaft 828 and arranged below the first link 912, and the gas spring 920 connected to the third shaft 826 and the second shaft 824 wherein the first link 912 may extend from the third shaft 826 toward the first shaft 822 such that one end thereof may be connected to the compensation shaft 810.

In addition, although not illustrated in FIG. 10, as illustrated in FIG. 9, the gravity compensation control device 700 may further include the third link 916 connected to the first shaft 822 and the second shaft 824, and the fourth link 918 connected to the third shaft 826 and the fourth shaft 828.

As illustrated in FIGS. 9 and 10, the disclosure is to improve issues of an existing passive gravity compensation control device using a gas spring. According to the disclosure, when an additional compensation force is required, for example, when the weight of a control panel or the like increases or a lift movement angle increases, multiple compensation forces may be provided through compression of the elastic member 1030 by adding the elastic member 1030 without replacing a main elastic member, for example, the gas spring 920, and thus, the degree of freedom of compensation gravity increases compared to existing devices.

The gravity compensation control device 700 according to the disclosure does not require replacement of the gas spring 920, which is the main elastic member, or addition of a separate compensation device, thus does not required reliability and durability testing, and does not have any issue of deterioration in usability thereof, such as the need to adapt to changes in operating force.

As illustrated in FIGS. 7 to 10, the gravity compensation control device 700 according to the disclosure may include the bearing unit 1010.

The bearing unit 1010 may include the bearing shaft 1012 arranged in the first mounting unit 800, and the bearing 1014 coupled to the bearing shaft 1012. The bearing unit 1010 may rotate about the bearing shaft 1012 or move along a particular path, and may simultaneously rotate and move.

A guideline 1040 is formed on the first mounting unit 800 of the gravity compensation control device 700 according to the disclosure, and serves to provide a path along which the bearing unit 1010 moves.

That is, the bearing unit 1010 is capable of at least one of rotating and moving along the guideline 1040 arranged in the first mounting unit 800.

As illustrated in FIG. 9, a nut 1050 may be coupled to one end of the elastic member 1030 of the disclosure. By combining the nut 1050 for force adjustment with the end of the elastic member 1030, it is possible to additionally change the pressure of a compensation material, and thus increase the compensation force and fine-tune the operating force.

According to another embodiment, caps may be coupled to both ends of the elastic member 1030. The cap coupled to one end of the elastic member 1030 serves to support the bearing unit 1010, and the cap coupled to the other end may be coupled to the nut 1050 to receive a force. Referring to FIG. 8, in case that the weight compensation device 1000 includes a plurality of elastic members 1030, a guide link 1060 may be disposed at the other end of the plurality of elastic members 1030. The guide link 1060 can guide the plurality of elastic members 1030 to operate together.

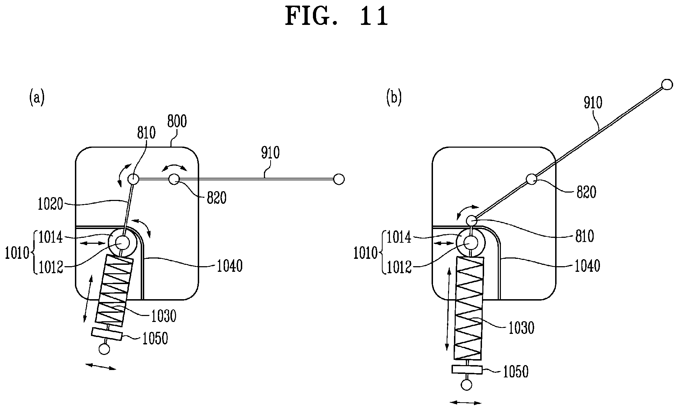

FIG. 11 is a diagram for describing an operation of a gravity compensation control device according to an embodiment.

As illustrated in FIG. 11, the bearing unit 1010 according to the disclosure is capable of at least one of rotating and moving along the guideline 1040.

As illustrated in (a) and (b) of FIG. 11, the first shaft 822 is rotatable, and the first link 912 is rotatable about the first shaft 822 such that both left and right ends thereof move up, down, left, and right. The compensation shaft 810 is rotatable and movable, and (b) of FIG. 11 illustrates that the compensation shaft 810 moves downward with respect to the first shaft 822, which may mean that the position of the control panel or the like on the right end has been raised.

That is, it may be seen that the elastic member 1030 is compressed in (a) of FIG. 11, whereas the length of the elastic member 1030 increases as the elastic member 1030 is uncompressed (b) of FIG. 11.

The gravity compensation control device 700 according to the disclosure performs gravity compensation by using the elastic member 1030 that is movable and rotatable inside the guideline 1040, and a resulting compressive force of the bearing unit 1010, as illustrated in (a) and (b) of FIG. 11.

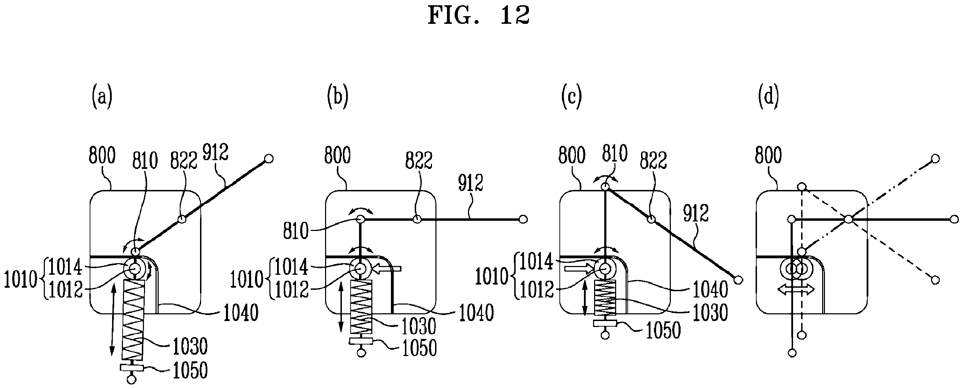

FIG. 12 is a diagram for describing an operation of a gravity compensation control device according to an embodiment.

FIG. 12 illustrates each process of FIG. 11 in more detail. (a), (b), and (c) of FIG. 12 illustrate cases where the left side of the first link 912, that is, the compensation shaft 810, moves downward, is horizontal, and moves upward with respect to the first shaft 822, respectively, and (d) of FIG. 12 illustrate the cases of (a), (b), and (c) in one view.

As the right end of the first link 912 vertically moves according to a change in the weight applied to the right end, the first link 912 is tilted. The compensation shaft 810 of the first link 912 is moving downward when transitioning from the state illustrated in (a) of FIG. 12 to the state illustrated in (b) of FIG. 12 and then the state illustrated in (c) of FIG. 12, which means that the weight applied to the right end is increasing.

When a lift is combined on the right end, the above process corresponds to a process in which the lift ascends, then moves to a middle position, and then descends.

In the disclosure, as the bearing unit 1010 is illustrated as being rotatable and movable along the guideline 1040, when transitioning from the state illustrated in (a) of FIG. 12 to the state illustrated in (b) of FIG. 12, the lift at the top moves to the middle position, and at this time, the elastic member 1030 is being compressed, and the bearing unit 1010 is moving to the left.

In addition, when transitioning from the state illustrated in (b) of FIG. 12 to the state illustrated in (c) of FIG. 12, the lift descends from the middle position, thus, the elastic member 1030 is further compressed to the maximum, and the bearing unit 1010 moves to the right again in the state illustrated in (b) of FIG. 12 and thus moves to its original position.

The bearing unit 1010 that is movable only inside the guideline 1040 is movable upward to a limited position, and thus can no longer move upward, and thus, as illustrated in (b) and (c) of FIG. 12, the elastic member 1030 coupled to the bearing unit 1010 is compressed.

The bearing unit 1010 according to the disclosure is rotatable and movable along the guideline 1040, whereas a component of a related-art structure corresponding to the bearing unit 1010 is fixed to a particular position and thus immovable.

Thus, the related-art structure has a disadvantage in that a change in the angle formed by the elastic member 1030 due to a lift ascending or descending is large, and thus, a large space is occupied due to the angle change. On the other hand, the gravity compensation control device 700 according to the disclosure has a structure in which the bearing unit 1010 is also horizontally movable inside the guideline 1040, and thus, there is almost no change in the angle of the elastic member 1030, which has the effect of minimizing the angular movement of the elastic member 1030.

That is, as illustrated in (d) of FIG. 12, it may be seen, from the inclination of the elastic member 1030 according to ascent and descent of the lift, that the elastic member 1030 moves while maintaining an almost vertical posture, and thus, there is almost no change in the angle. As illustrated in FIG. 12, in the disclosure, the angular movement of the elastic member 1030 may be minimized when the lift ascends or descends, and thus, only a small space is occupied, which has the effect of increasing the usability of the internal space.

That is, in the disclosure, the elastic member 1030 may be used in a narrow space, which has the effect of facilitating control for a required angle.

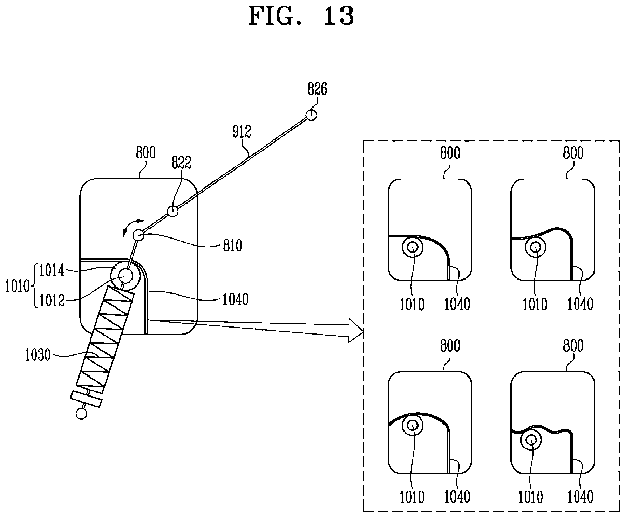

FIG. 13 is a diagram for describing a gravity compensation control device according to another embodiment.

The bearing unit 1010 in the disclosure is movable along the guideline 1040 formed in the first mounting unit 800, and as illustrated in FIG. 13, the shape of the guideline 1040 may include at least one of a straight line and a curve, and the shape of the curve is not limited to a particular shape.

In that the position of the bearing unit 1010 is controlled according to the shape of the guideline 1040, the shape of the guideline 1040 is a factor that affects pressure and angle control, and the elastic force of the weight compensation device 1000 may be adjusted by adjusting the shape of the guideline 1040.

In addition, although not illustrated in the drawings, according to an embodiment, in the entire length of the first link 912, the length between one end of the first link 912, that is, the compensation shaft 810, and the first shaft 822 may be less than the length between the compensation shaft 810 and the other end (i.e., the third shaft 826) of the first link 912, that is, the portion to which a load is applied.

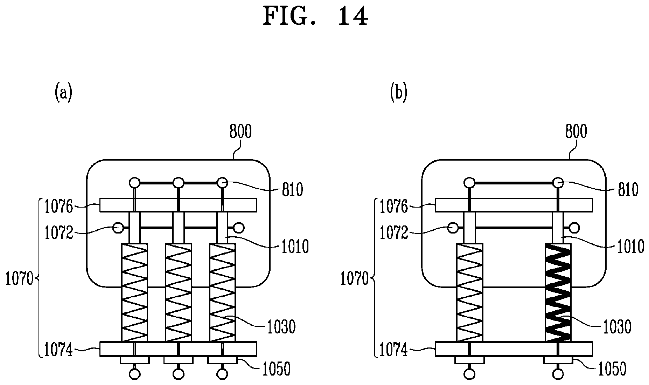

FIG. 14 is a diagram for describing a gravity compensation control device according to another embodiment.

In FIG. 14, a plurality of weight compensation devices 1000 according to the disclosure may be provided, and as illustrated in (a) of FIG. 14, a connection unit 1070 connecting the plurality of weight compensation devices 1000 to each other may be included.

According to an embodiment, the connection unit 1070 may include a first connection unit 1072 connecting a plurality of bearing units 1010 to each other, a second connection unit 1074 connected to a plurality of nuts 1050, and a third connection unit 1076 connecting a plurality of weight compensation devices 1000 to the first mounting unit 800. At least a portion of the connection portion 1070 may perform a function of guiding the plurality of weight compensation devices 1000 to operate together. For example, the second connection unit 1074 may be used as a guide link 1060 that guides the plurality of elastic members 1030 to operate together.

FIG. 14 illustrates all of the first connection unit 1072, the second connection unit 1074, and the third connection unit 1076, but the weight compensation device 1000 according to the disclosure may include at least one of the components of the connection unit 1070, and the connection structure is not limited to the structure illustrated in FIG. 14, and may be any structure that allows connection between the components of the weight compensation device 1000.

The plurality of weight compensation devices 1000 may include a plurality of elastic members 1030, respectively, and the plurality of elastic members 1030 may all be the same.

The respective elastic members provided in the plurality of weight compensation devices 1000 may have different elastic forces.

According to an embodiment, as illustrated in (b) of FIG. 14, the plurality of elastic members 1030 may differ from each other in thickness, and for example, may differ from each other in at least one of outer diameter, length, thickness, and elastic coefficient.

In the disclosure, the compensation force may be adjusted by changing the number of weight compensation devices 1000 and the type of elastic member 1030.

Of course, the compensation force may be adjusted by changing a configuration of the elastic member 1030, which affects the characteristics of the elastic member 1030, in addition to the outer diameter, length, thickness, and elastic coefficient described above.

The disclosed embodiments are described above with reference to the accompanying drawings. It will be understood by one of skill in the art that various changes in form and details may be made therein without departing from the spirit and essential features of the disclosure. The disclosed embodiments should be considered in descriptive sense only and not for purposes of limitation.

A gravity compensation control device and an ultrasonic diagnostic device including the same according to the disclosure may adjust a compensation force without deteriorating usability, thereby increasing the degree of freedom of compensation gravity compared to existing devices.

The effects of the disclosure are not limited to those described above, and other effects not described herein may be clearly understood by one of skill in the art from the present specification and the accompanying drawings.

It should be understood that embodiments described herein should be considered in a descriptive sense only and not for purposes of limitation. Descriptions of features or aspects within each embodiment should typically be considered as available for other similar features or aspects in other embodiments. While one or more embodiments have been described with reference to the figures, it will be understood by those of ordinary skill in the art that various changes in form and details may be made therein without departing from the spirit and scope of the disclosure as defined by the following claims.

## Claims

1. A gravity compensation control device comprising:
a first mounting unit (800);
a rotatable link (910), one end of which is connected to a compensation shaft (810) arranged in the first mounting unit and another end of which is vertically movable by a weight applied thereto, wherein a portion between the ends is supported by a fixed shaft (820) arranged in the first mounting unit, such that the link is rotatable about the fixed shaft; and
a weight compensation device (1000),
wherein the weight compensation device (1000) comprises:
a bearing unit (1010) comprising a bearing shaft (1012) arranged in the first mounting unit, and a bearing (1014) coupled to the bearing shaft;
an auxiliary link (1020), one end of which is connected to the compensation shaft and the other end of which is connected to one side of the bearing unit; and
an elastic member (1030) coupled to another side of the bearing unit and arranged in a lengthwise direction of the auxiliary link.

2. The gravity compensation control device of claim 1, wherein the bearing unit is capable of at least one of rotating and moving.

3. The gravity compensation control device of claim 1 or 2, wherein the bearing unit is capable of at least one of rotating and moving along a guideline arranged in the first mounting unit.

4. The gravity compensation control device of any one of claims 1 to 3, wherein a nut (1050) is coupled to one end of the elastic member.

5. The gravity compensation control device of any one of claims 1 to 4, further comprising a guide link (1060) at one end of the elastic member.

6. The gravity compensation control device of any one of claims 1 to 5, wherein the weight compensation device comprises a plurality of weight compensation devices.

7. The gravity compensation control device of claim 6, further comprising a connection unit (1070) connecting the plurality of weight compensation devices to each other.

8. The gravity compensation control device of claim 6, wherein the respective elastic members arranged in the plurality of weight compensation devices have different elastic forces.

9. The gravity compensation control device of any one of claims 1 to 8, wherein the compensation shaft is rotatable.

10. The gravity compensation control device of any one of claims 1 to 9, further comprising a first mounting unit having a first shaft (822) and a second shaft (824) arranged therein, and a second mounting unit that is vertically movable and has a third shaft (826)and a fourth shaft arranged therein,
wherein the rotatable link is at least one of a first link (912) connected to the first shaft and the third shaft, a second link (914) connected to the second shaft and the fourth shaft and arranged below the first link, a third link (916) connected to the first shaft and the second shaft, and a fourth link (918) connected to the third shaft and the fourth shaft.

11. The gravity compensation control device of claim 10, further comprising a gas elastic member (920) connected to the third shaft and the second shaft.

12. An ultrasonic diagnostic device (1100) comprising the gravity compensation control device of any one of claims 1 to 11, the ultrasonic diagnostic device further comprising:
a main body (1200) connected to the first mounting unit; and
a control panel (1300) connected to a connection frame (1400) to be movable up, down, left, right, or diagonally with respect to the main body,
wherein the connection frame comprises the gravity compensation control device.
